Europäisches Patentamt

**European Patent Office**

Office européen des brevets

(11) Publication number: **0 189 381**
A2

(12) # EUROPEAN PATENT APPLICATION

(21) Application number: **86830007.0**

(22) Date of filing: **14.01.86**

(51) Int. Cl.⁴: **H 04 N 5/33**

(30) Priority: **14.01.85 IT 4754285**

(43) Date of publication of application: **30.07.86**
**Bulletin 86/31**

(84) Designated Contracting States: **AT BE CH DE FR GB LI LU NL SE**

(71) Applicant: **ELETTRONICA S.p.a., Km. 13+700 Via Tiburtina, I-10131 Rome (IT)**

(72) Inventor: **Salcito, Giuliano, 34, Via Monte Gran Paradiso, I-00010 Colleverde di Guidonia Rome (IT)**

(74) Representative: **Cioncoloni, Enrico, Dr., Studio Consulenza Brevetti 76, Via F. Turati, I-00185 Rome (IT)**

(54) **Apparatus for contact thermography with acquisition of images by means of a colour telecamera, digital conversion and processing of the output digital data.**

(57) Apparatus for contact thermography comprising a thermographic liquid crystal plate (11) so be placed upon the surface of a body to be examined, so that the image evidenced by the different colours taken on point-by-point by the liquid crystals because of the different temperatures may be shooted by a colour telecamera (12) arranged as desired by means of an auxiliary pointing device (13). The composed video output is passed to a first converter (14) to convert the image into digital data relating, separately, to the fundamental colours, namely red, green, blue, each one corresponding to a pixel, i.e. to an unit of the digital coded image further qualified by the address with its co-ordinates, luminosity and chrominance. The entire data are then passed to a pre-processor (15) as second converter to convert the chrominance code into temperature values and to a processor (16) to carry out following functions: to draw out the information contained in single images; to compose the images of same subject obtained however by using different thermographic plates; to compare the images of same subject obtained however at different times.

# APPARATUS FOR CONTACT THERMOGRAPHY WITH ACQUISITION OF IMAGES BY MEANS OF A COLOUR TELECAMERA, DIGITAL CONVERSION AND PROCESSING OF THE OUTPUT DIGITAL DATA

The invention relates to an apparatus for contact thermography and particularly to an apparatus wherein the images evidenced by the different colours taken on point-by-point by the liquid crystals of a thermographic plate when placed upon the surface of a body to be examined are shooted by a colour telecamera arranged as desired by means of an auxiliary pointing device, the composed video output being then passed to a first converter to convert the images into digital data. Each digital datum corresponds to a "pixel", i.e. to a unit of the digital coded image, further qualified by the address with its co-ordinates, luminosity and chrominance. The data as a whole are then sent to a pre-processor to convert the chrominance code into temperature values and later on to a processor, the scope being to carry out following functions: to draw out the information contained in single images; to compose the images of a like subject obtained however by using different thermographic plates; to compare images of a like subject obtained however at different times.

The thermography, the scope of which is to evidence the distribution of the temperature on the surface of a body to be examined in detail, has from many years found a particular application to study pathologies which are producing an anomalous distribution of the temperature, in order to carry out a valid physiological and diagnostic search thereabout. The noting and quantitative evaluation of such anomalous distribution have been and are updating considered of a particular importance by using infrared radiations emitted from the body surface, and exploiting suitable films or plates sensitive to such radiations, as well as proper telethermocamera fit for detecting these latter at a remote position.

However, the telethermographic process requires the use of apparatus of considerable expense, so that in the last years the contact thermography has been developed to take advantage of the thermographic analysis as a dynamic method to perceive those thermic modifications which are associated to pathologic processes on the surface of the examined body, the study and evaluation of same being obviously important for the diagnosis and consequent prognosis or watching, particularly when such analysis may prevent or diagnose some growing worses.

The contact thermography makes use of the important feature of liquid crystals as a mesomorphic phase of the material, with the mechanical features of the liquids and the features of the crystals. However, it makes above all use of the proper features of some of these crystals with :selective reflection of white light and dependance of the resulting colour on the temperature when they are exposed to predetermined lighting and observation. On the other hand, some mixture of liquid crystals, e.g. those of cholesterol, permit the pointing out of the thermic distribution on the examined surface.

The liquid crystals in their updating use for contact thermography take the shape of microballs encapsulated within a transparent polymer to form plates which may be differentiated on the ground of their thermic features, and are protected as much as possible against thermo-optical alterations in order to minimize ageing phenomenons.

According to the prior art, this dynamic method of contact thermography, while being economically advantageous in respect to the examination through infrared radiations emitted by single points of the tested surface, as suggested by the telethermografic method, evidences, between other things, two disadvantages: a) allows practically only a qualitative analysis; b) permits a diagnosis as dependent on the subjective competency of the operator. Furthermore it is to be added that also when the best attention is paid and a suitable selection is made to perform the encapsulation of the microballs of liquid crystals, the thermo-optical features of the prepared plates cannot be considered inalterable for a very long time, so that the checking and recalibration of the plates is necessary.

The invention as claimed is intended to remedy these defectivenesses and drawbacks of the apparatus suggested and/or applied in accordance with the prior art. It solves the problem of how to create a reliable apparatus to carry out a physiological and diagnostic search, particularly in the case of pathology concerning the mamma ... also with qualitative evaluations and expenses lower than for telethermographic examinations. Main object of the invention is to reach such finality by digitizing the images obtained by a colour telecamera for contact thermography, and pass the resulting digital data to a personal computer to process them like the propositions and display suggested for a telethermographic process.

It is therefore an object of the invention to use plates for contact thermography and shoot by a colour telecamera the image shown by the liquid crystals in accordance with the colour code, to indicate the temperature in different points of the plate and convert the composed video into digital data by a first composed video/RGB converter, in order to draw out, separately, the fundamental component colours R-G-B (Red-Green-Blue) of the chrominance-luminance vector, as well as the horizontal and vertical synchronisms to be used for storing the digital data.

It is another object of the present invention to use a converter of the temperature code, to receive the output digital data of said first converter which are indicative of the digitized image and pass them to a processor through a suitable interface circuital system comprising shift registers, dynamic RAM and three-state buffers, as well as through a pre-processing furction. The processor may be of the type used for telethermography, or suitable to exploit the tridimensionality because the programming of same is actually easy.

The invention will be described further, by way of example, with reference to the accompanying drawings wherein:

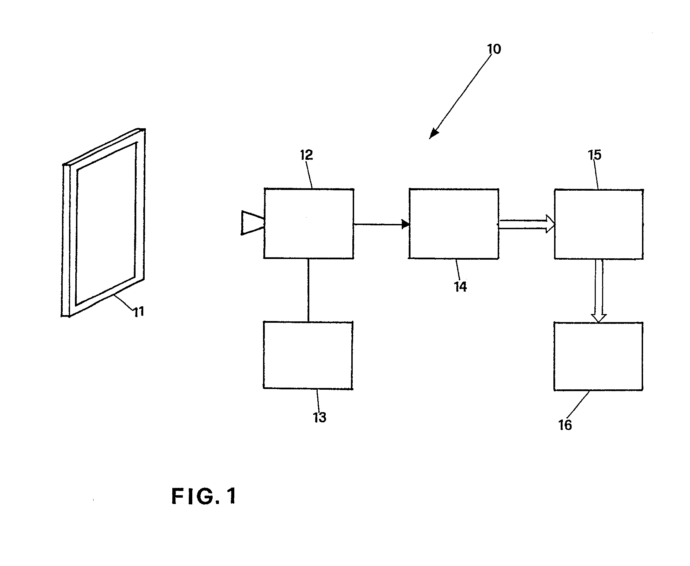

Fig.1 is a block diagram of an apparatus illustrating a preferable mode of carrying out the method of contact thermography in accordance with this invention;

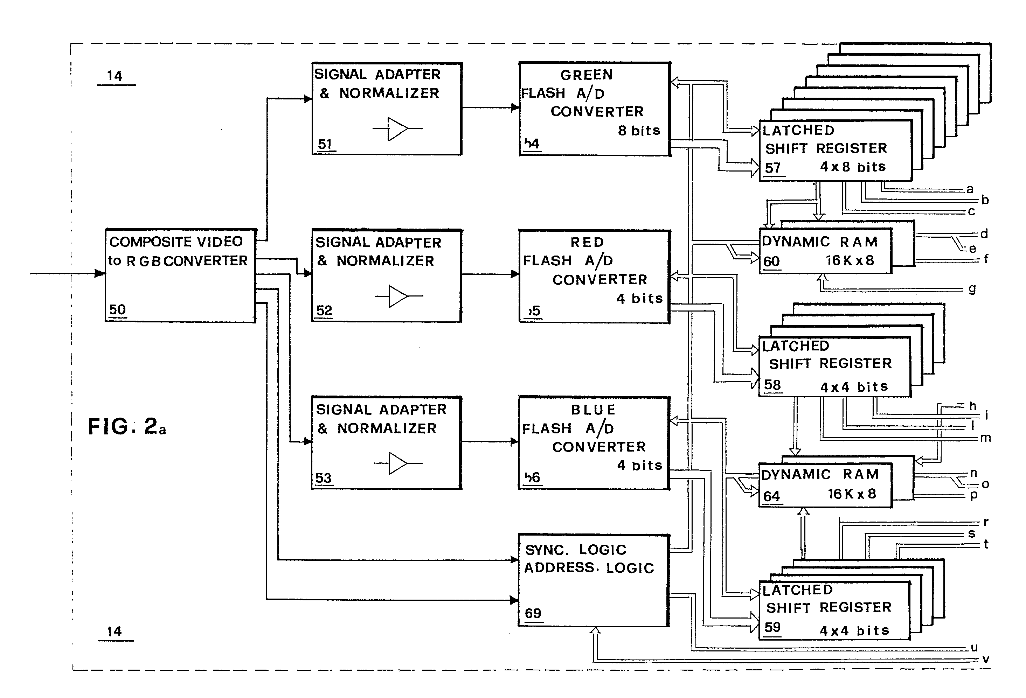

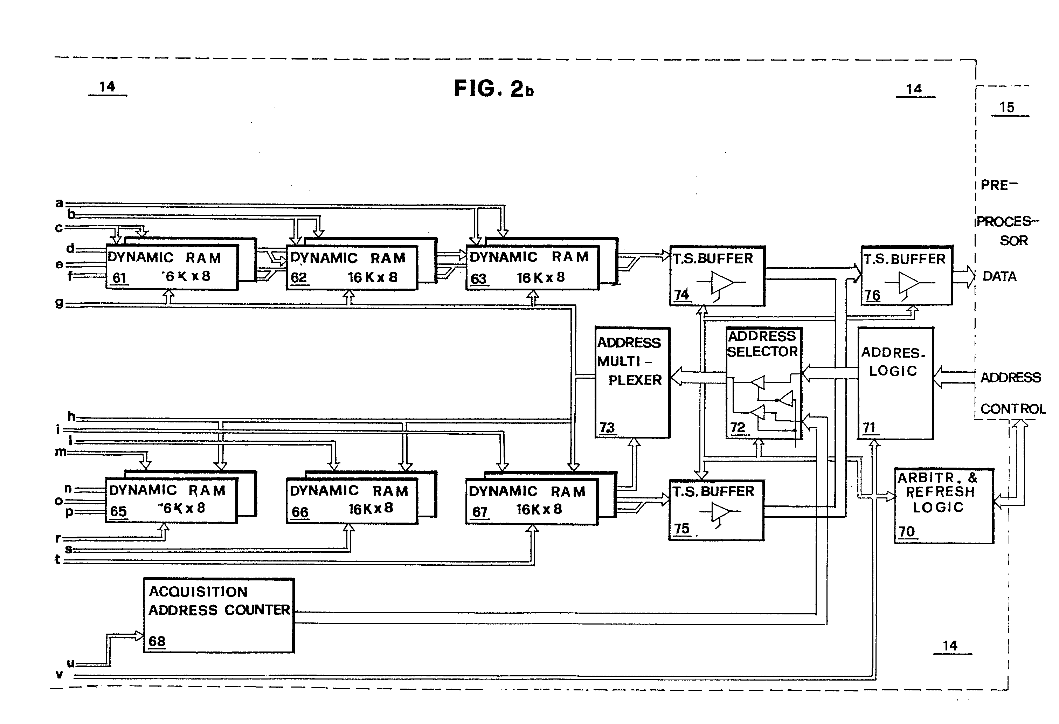

Figs.2a and 2b form, in the whole, a detailed representation of a circuital system the components of which carry sequentially out the operative phases which go on from the conversion of the composed video resulting from the shooting by a colour telecamera into digital data, up to the separation of the fundamental colours; a first transitory storage of the digital data of the image resulting by the shoot and horizontal and vertical synchronisms used to store same with a correct addressing; the sending of information to a pre-processor;

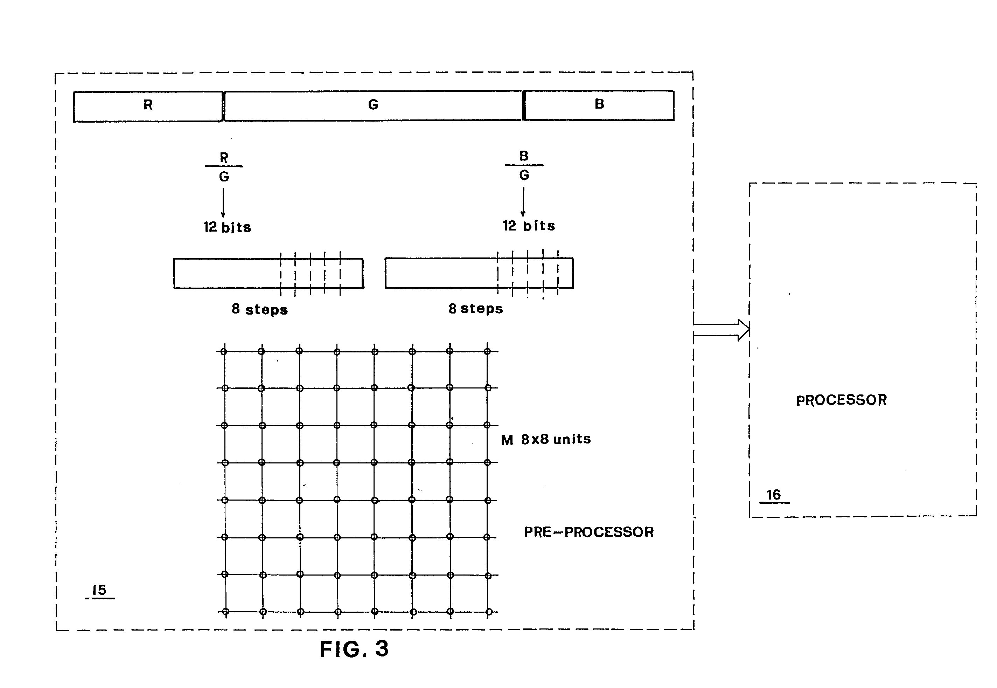

Fig.3 is a schematic view of a pre-processor as conversion algorithm RGB/chrominance and chrominance/temperature, with formation of a matrix the units of which correspond to a temperature beam evidenced by the plate for contact thermography and are passed to a processor to be processed by carrying on the foreseen functions of the apparatus in accordance with the invention and permitted by the particular features of the components of that circuital system and pre-processor.

Turning now to the drawings and first of all to Fig.1 , a block system is provided to show schematically an apparatus 10 comprising:

- a liquid crystal plate 11 which may be placed into contact with the surface of a body to be examined and thus used for contact thermography;

- a colour telecamera 12 which may be arranged as desired by means of an auxiliary device 13 in order to shoot the image formed through the liquid crystals of plate 11;

- a circuital system 14 to convert the composed video into digitized fundamental colours R-G-B of the chrominance-luminance vector, to draw separately out the digitized data of component colours Red, Green, Blue as well as the horizontal and vertical synchronisms to be used for storing the digitized data and then obtaining a whole of digital data, each one corresponding to a "pixel" or unit of the digitized image, qualified also by the address with its co-ordinates, luminosity and chrominance, this latter being intended as a relative or absolute intensity of the three fundamental colours;

- a pre-processor 15 to draw out the relative intensity from the chrominance datum and convert same into temperature values to be compared with the colour-temperature scale of the used thermographic plate;

- a processor 16 to process the output information of converter 15.

To better understand the innovating features of the present invention a detailed description of same is reported hereafter, referred particularly to the detailed circuital system 14 the several components of which are illustrated in two sections to be interconnected, namely Fig.2a and Fig.2b.

The composed video coming out from the telecamera 12 passes through a first composed video/RGB converter indicated by the reference numeral 50 in Fig.2a, the scope of which is to provide the components R-G-B of the chrominance-luminance vector, separately from each other, as well as the horizontal and vertical synchronisms, this latter being provided as a component for the storage of the digital converted data and indicated in Fig.2a by reference numeral 69.

As shown in Fig.2a, the analogic signal received by converter 50 is sent to three amplifiers 51, 52, 53 as SIGNAL ADAPTER & NORMALIZER for R-C-B, respectively, the input to the converters being so matched with a suitable impedance and regulated amplification, that the entire dynamic may be centered.

FLASH A/D CONVERTERS 54, 55, 56 receive the output signal of respective channel, each converter being operated according to a particular ECL (Emitter Coupled Logic) technique in order to carry out the conversion within a real time. As shown in the representative component blocks of FigS.2a and 2b, a resolution of 4 bits is required for the red and blue colours, while the resolution of 8 bits is required for the green colour, such requirement being in accordance with the central position of the green colour in the spectrum.

The outputs of converters 54, 55, 56 pass to LATCHED SHIFT REGISTERS 57, 58, 59, respectively for a temporary storage and formation of messages to send to the memories. DYNAMIC RAM 60 to 63 and 64 to 67 (see also Fig.2b) contain the information relating to the entire image addressed during the acquisition by an ACQUISITION ADDRESS COUNTER component 68.

The synchronism and clock of acquisition are drawn out from said SYNC.LOGIC-ADDRESS.LOGIC 69 (Fig.2a) which keeps into account the video sychronisms (H SYNC.-V SYNC.) with refresh control assigned to the ARBTR.& REFRESH LOGIC component indicated by the reference numeral 70. This refresh circuit intervenes when the image memories are operated directly by the personal computer 15-16 of the apparatus, to carry out the transfer into processor 16 through pre-processor 15 and provide also the exchange of the DATA and ADDRESSING BUS from the personal computer to the circuits of ADDRESS SELECTOR component 72. It is to point out that the total addressing capacity of the personal computer is extended from 16 to 20 bits by the logic of ADDRESS. LOGIC circuit 71 in order to cover the dynamic RAM capacity.

As shown in Fig.2b, DYNAMIC RAM 16 K x 8 are provided and the addressing of same for line and column is drawn out from the circuits of an ADDRESS MULTIPLEXER 73.

When the computer 15-16 asks for data from the memo-ries, the addressing is to be arranged beforehand through inner address bus by activating the digital multiplexer 73 as well as the refresh circuit to be synchronized with the inner reading. The data bus is to be activated in those three-state buffers which have been forbidden during the acquisition.

As premised and shown in Figs.2b and 3, a pre-processor 15 is provided as conversion algorithm RGB to relative chrominance and relative chrominance to temperature of the used termographic plate; that is to say, a preliminary processor to draw out the chromatic scales by normalizing the component R in respect to G and then the component B in respect to G. The result of this operation involves two words of 12 bits, each one giving the possibility of selecting 8 steps. A conversion scale is in this manner created in the form of a matrix M of 8x8 = 64 units as shown schematically in Fig.3.

It will be understood that, on the ground of this feature it is also possible to compose temperature scales more extended than those actually available, by using more matrixes when the employed plates have temperature intervals adjacent to each other or partially superimposed. It is also possible to verify and recalibrate the matrixes by acquiring a black body as a reference of calibration.

Results will thus be obtained which concern: a suitable/of interval measurable temperatures; a calibrated scale; and the consequent conversion of the acquired colours into reliable temperature data. On the other hand conversion tables may be stored in pre-processor 15 on semiconductor digital permanent memories (e.g. EPROM, PROM, ROM and the like) or also on magnetic digital permanent memories (e.g. of the tape or disk type, either "floppy", or "hard", or "bubble memory", as magnetic bubble memory). The selection of the table is made through identification of the used plate, and the calibration is made periodically by acquiring the reference to a known temperature, and storing on permanent memory the value of the corresponding calibration.

In Fig.3 the processors 16 is shown as a dashed block only, referred to the last component of the apparatus which, though being by itself a novel processing means to be claimed in accordance with the present invention, carries out functions according to known principles, namely: to draw out the information contained in single images; to compose images of the same subject obtained however by different thermographic plates with a consequent increase of the temperature dynamic; to compare images of the same subject obtained however at different times. With regard to this, it is important to evidence that, for example, in the case of contact thermographic analysis relating to the mamma,.. further to the usual display of image/images from the right, left and front side, separately, for the right and left mamma:,. it is possible to add advantageously a tridimensional display programmed for the processor, as said above.

It is also important to note that by using an apparatus provided with the components better detailed in Figs.2a, 2b and 3 it is foreseen the use of some other thermographic plates in addition to those which are in sale. As a matter of fact, while by using thermographic plates available at present according to the prior art, a really skilled operator may deduce immediately subjective evaluations on the ground of his own capacity of visual observation and give an opinion based on clean changes of colours - usually from 3 to 8 different colours for the entire dynamic of temperatures - by employing an apparatus in accordance with the present invention it is possible the use of some other plates indicative of colour shades that a human eye could not resolve and word quantitavely. It would be understood by the skilled in the art that because of such possibility the thermic resolution allowed by an apparatus according to this invention is higher, and the obtainable results are after- all in keeping either for the diagnosis and prognosis, as well as for the watch.

A further consideration is to be made with regard to the shooting by colour telecamera 12, i.e. that by the use of a suitable auxiliary pointing device 13 there is not a remarkable limitation of distance between plate 11 and telecamera 12 of an apparatus 10, differently from the limitation required when an apparatus for contact thermography in accordance with the prior art is employed wherein such distance is defined by the possible extension of the apparatus arm used for a thermografic examination and photographic shooting.

0189381

- 1 -

Claims :

1.   Apparatus for contact thermography provided with liquid crystal plates to be placed upon the surface of a body to be examined in order to: shoot, by means of a colour telecamera, the images of the distribution of temperatures through the different colours taken on point-by-point by the liquid crystals of the thermographic plate; digitize the output data; convert the chrominance code; and pass to a personal computer the results to be processed; c h a r a c t e r i z e d  in that it comprises:

- a colour telecamera (12) with auxiliary pointing device (13) to shoot the image of the surface of a body to be examined, shown on a thermographic plate (11) by exploiting the different colours and tonalities taken on by the liquid crystals;

- a circuital system (14) a first component of which (50) converts the output composed signal of said colour telecamera (12) into digital data to draw out, separately, the component colours Red-Green-Blue (RGB) of the chrominance-luminance vector, as well as the horizontal and vertical synchronisms for the storage of the converted digital data, said component colours RGB being amplified by SIGNAL ADAPTER & NORMALIZER (51, 52, 53), respectively and sent to FLASH A/D CONVERTER (54, 55, 56), respectively and then to LATCHED SHIFT REGISTER (57, 58, 59), respectively for a temporary storage, and formation of messages to be passed to DYNAMIC RAM (60 to 63) and (64 to 67) containing the image in its whole and addressed during the acquisition through a component (68), said horizontal and vertical synchronisms being provided in a component circuit (69); a further REFRESH LOGIC circuit (70) as well as ADDRESS LOGIC, SELECTOR, and MULTIPLEXER (71, 72, 73), respectively and THREE-STATE BUFFER (74, 75, 76) being also provided and so connected to complete said circuital system (14) in such a manner that the output digital data may be passed to a pre-processor (15) for a preliminary processing of same;

- a conversion algorithm R G B to chrominance and chrominance to temperatures of the used thermographic plates, which forms such PRE-PROCESSOR (15) to draw out the chromatic scales by normalization of component (R) in respect to (G) and then of component (B) in respect to (G), two words of 12 bits resulting from said operation each one of them giving the possibility of selecting 8 steps, so that a matrix (M) of 8 x 8 = 64 units is provided;

- a PROCESSOR (16) to process the output data of said pre-processor (15).

2. An apparatus as claimed in Claim 1, c a r a c t e r i z e d in that said converter (15) converts the colours acquired through said converter (14) into reliable temperature data; and that the conversion scales in the form of tables (matrixes M) may be stored on permanent digital memories of a semiconductor type, e.g. EPROM, PROM, ROM, or of a magnetic type.

3. An apparatus as claimed in Claim 1, c a r a c t e r i z e d in that said processor (16) stores the digital data of temperature acquired also to carry out following functions:

- to draw out the information contained in single images;
- to compose and integrate the images of same subject obtained however by different thermographic plates in different positions;
- to compare therebetween images of same subject obtained however at different times;

- to make use of temperature intervals more extended than by a single plate.

4.    An apparatus as claimed in Claim 1 c h a r a c t e r i z e d in that the composition of images coming out by means of said processor (16) is also possible for a tridimensional display of same.

5.    An apparatus as claimed in Claims 1, 3, 4 c h a r a c t e r i z e d  in that it permits the use of some other thermographic plates in addition to those usually used for contact thermography, such other plates being suitable to supply colour shadings which are generally not perceptible through the eye of a skilled observer.

6.    An apparatus as claimed in Claims 1 and 4 c h a r a c - t e r i z e d  in that said processor (16) permits to realize both qualitative and quantitative analysis of the thermogram coming out from said pre-processor (15) as chrominance converter of said output digital data of said chrominance/temperature converter (14).

0189381

114

FIG. 1

FIG. 2a

14

COMPOSITE VIDEO to R G B CONVERTER
50

SIGNAL ADAPTER & NORMALIZER
51

SIGNAL ADAPTER & NORMALIZER
52

SIGNAL ADAPTER & NORMALIZER
53

GREEN FLASH A/D CONVERTER
8 bits
54

RED FLASH A/D CONVERTER
4 bits
55

BLUE FLASH A/D CONVERTER
4 bits
56

SYNC. LOGIC ADDRESS. LOGIC
69

LATCHED SHIFT REGISTER
57    4 x 8 bits

DYNAMIC RAM
60    16 K x 8

LATCHED SHIFT REGISTER
58    4 x 4 bits

DYNAMIC RAM
64    16 K x 8

LATCHED SHIFT REGISTER
59    4 x 4 bits

a
b
c
d
e
f
g
h
i
l
m
n
o
p
r
s
t
u
v

14

0189381

# FIG. 2b

14

14

15

PRE-

PROCES-
SOR

DATA

a
b
c
d
e
f
g

DYNAMIC RAM 61 16 K x 8

DYNAMIC RAM 62 16 K x 8

DYNAMIC RAM 63 16 K x 8

T.S.BUFFER 74

T.S.BUFFER 76

ADDRESS
MULTI-
PLEXER
73

ADDRESS
SELECTOR
72

ADDRES.
LOGIC
71

ADDRESS

CONTROL

h
i
l
m

n
o
p

DYNAMIC RAM 65 16 K x 8

DYNAMIC RAM 66 16 K x 8

DYNAMIC RAM 67 16 K x 8

T.S. BUFFER 75

ARBITR. &
REFRESH
LOGIC
70

r
s
t

ACQUISITION
ADDRESS COUNTER
68

14

u
v

0189381

FIG. 3